# NOUVEAU FASCICULE DE BREVET EUROPEEN

(11) **EP 2 272 836 B2**
(45) Date de publication et mention de la décision concernant l'opposition: **17.02.2021**
(45) Mention de la délivrance du brevet: 14.01.2015
(21) Numéro de dépôt: 10168406.6
(22) Date de dépôt: 05.07.2010
(51) Int. Cl.: C07D 285/125

(54) **Procédé de préparation de bis-DMTD**
Verfahren zur Herstellung von Bis-DMTD
Process for the preparation of bis-DMTB

(30) Priorité: 06.07.2009 FR 0903323
(43) Date de publication de la demande: 12.01.2011
(73) Titulaire: MLPC International, 40370 Rion-Des-Landes (FR)
(72) Inventeur: Coucharriere, Carole, 40400, Carcen Ponson (FR); Aubert, Thierry, 64000, Pau (FR); Bonhomme, Dominique, 40130, Capbreton (FR); Ehlinger, Jean-Yves, 40100, Dax (FR)
(74) Mandataire: Casalonga, Axel

(56) Documents cités:
- EP-A2- 0 135 152
- DE-B1- 958 650
- US-A- 3 087 932
- US-A- 3 087 932
- US-A- 4 599 425
- US-A1- 2006 168 741
- US-A1- 2006 168 741
- E. ZIEGELE: "Über Thiobiazoldithiol" JOURNAL FÜR PRAKTISCHE CHEMIE, 1899, VOLUME 60, ISSUE 1 (P 40-51), 10 juillet 1899 (1899-07-10), XP002570626
- DATABASE WPI Week 200868 Thomson Scientific, London, GB; AN 2008-L53199 XP002570627 & CN 101 096 366 A (CHINA PETRO-CHEM CORP) 2 janvier 2008 (2008-01-02) -& CN 101 096 366 A (CHINA PETROLEUM & CHEMICAL [CN]) 2 janvier 2008 (2008-01-02)
- ZIEGELE E: " ber Thiobiazoldithiol", JOURNAL FUER PRAKTISCHE CHEMIE., WILEY VCH, WEINHEIM., DE, vol. 60, no. 1, 10 July 1899 (1899-07-10), pages 40-51, DE ISSN: 1436-9966, DOI: 10.1002/prac.18990600103
- Traduction automatique de CN 101 096 366 (02.01.2008) & Traduction humaine de CN 101 096 366 (dep. le 10.03.2017)

## Description

La présente invention concerne un procédé amélioré de préparation de bis-(dimercaptothiadiazole) (ou bis-DMTD), plus particulièrement de 5,5'-dithiobis-(1,3,4-thiadiazole-2-thiol), également dénommé 5,5'-dithiobis-(1,3,4-thiadiazole-2(3*H*)-thione) sous sa forme tautomère, ledit procédé permettant une préparation avec des rendements améliorés et plus respectueuse de l'environnement.

La préparation de bis-DMTD est connue depuis de nombreuses années et est par exemple décrite dans la demande de brevet CN-A-101096366.

Ainsi, la synthèse de bis-DMTD est réalisée par oxydation de 2,5-dimercapto-1,3,4-thiadiazole (ou DMTD, CAS n° 1072-71-5), par exemple avec du peroxyde d'hydrogène, selon le schéma réactionnel suivant : 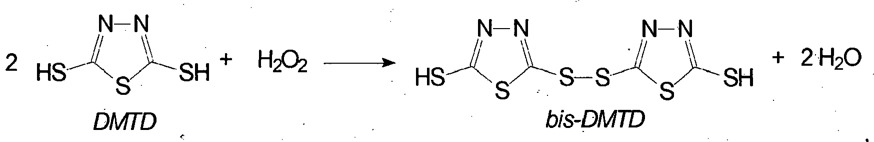 laquelle réaction pouvant également s'écrire, en faisant apparaître les formes tautomères :

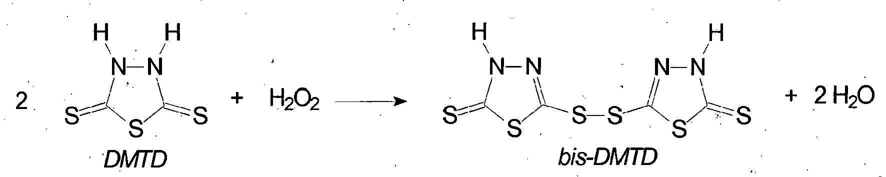

Cette réaction a fait l'objet d'un grand nombre d'études, comme par exemple celle publiée en 2006 dans la demande de brevet US 2006/0168741, et montrant, entre autres, la difficulté d'obtenir sélectivement le bis-DMTD, c'est-à-dire en minimisant la formation de dimère cyclique ou la formation d'oligomères, voire de polymères.

Toutefois, toutes ces études sont fondées sur l'utilisation, en tant que produit de départ, du 2,5-dimercapto-1,3,4-thiadiazole (DMTD).

Les travaux de E. Ziegele (dans Journal für praktische Chemie, (1989), 60, Issue 1, 40-51) montrent la nature des produits qui sont obtenus par réaction entre l'hydrazine et le sulfure de carbone (CS₂), en particulier le DMTD, et leurs comportements vis-à-vis de réaction d'oxydation ultérieure.

Dans le même esprit, le brevet US 3087932 présente la synthèse de DMTD à partir d'hydrazine et de CS₂, DMTD qui est directement engagé dans une étape d'oxydation, en association avec un thiol.

Le brevet US 4599425 présente quant à lui un procédé de synthèse du dimère cyclique du DMTD par réaction avec du peroxyde d'hydrogène en absence d'autre réactif.

Or il est bien connu que la réaction de synthèse de DMTD génère des effluents aqueux dans lesquels une quantité non négligeable de DMTD est dissous. La solubilité dans l'eau du DMTD est d'environ 8 g/L à 30 g/L, en fonction des conditions de température et de pH, ce qui entraîne une perte de rendement, mais aussi et surtout un traitement coûteux des effluents pour réduire leur teneur très importante en matière organique oxydable, mesurée par la Demande Chimique en Oxygène (DCO), qui est généralement de l'ordre de 15 g/L à 60 g/L environ.

En effet, la synthèse de DMTD est généralement réalisée à partir d'hydrazine et de sulfure de carbone, en solution aqueuse, selon le schéma réactionnel suivant : 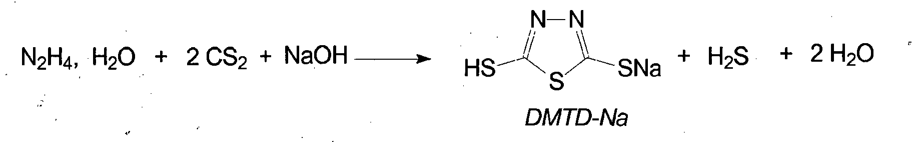 le sel de sodium du DMTD (DMTD-Na) étant ensuite acidifié en DMTD, au moyen d'une solution aqueuse acide, par exemple l'acide sulfurique en solution aqueuse.

Il reste donc aujourd'hui un besoin concernant un procédé de préparation de bis-DMTD qui soit sélectif et plus respectueux de l'environnement, par rapport aux procédés existants.

Ainsi, un premier objectif de la présente invention est de proposer un procédé de synthèse de bis-DMTD le plus sélectif possible, et avec des rendements les plus élevés possibles.

Un autre objectif de la présente invention est de proposer un procédé de synthèse de bis-DMTD le plus respectueux possible de l'environnement, et en particulier un procédé de synthèse dont les effluents générés sont plus faiblement chargés en produits organiques nécessitant par conséquent des traitements desdits effluents avec des coûts plus faibles.

La Demanderesse a maintenant découvert un procédé qui permet d'atteindre, en totalité ou au moins en partie, les objectifs précités, grâce au procédé de l'invention qui est détaillé dans la suite du présent exposé.

L'invention concerne un procédé de préparation de bis-DMDT, dans lequel l'intermédiaire de synthèse DMTD, obtenu en suspension dans l'eau (avec une solubilité d'environ 8 g/L à 30 g/L en fonction des conditions de température et de pH comme indiqué précédemment) n'est pas isolé, ce qui n'engendre donc pas d'effluents aqueux fortement chargés en DMTD générateur d'une DCO importante.

Le procédé de l'invention est ainsi réalisé dans un seul réacteur (« one pot reaction ») par introduction des matières premières (hydrazine et sulfure de carbone) et récupération du produit final, le bis-DMTD.

De manière surprenante par rapport à l'état de la technique où la synthèse « one-pot » n'est pas privilégiée, le procédé selon la présente invention, sans isolement du DMTD intermédiaire, conduit à l'obtention de bis-DMTD avec de bons rendements ainsi qu'une grande sélectivité. En outre, le produit final obtenu est d'une grande pureté, ce qui est rarement le cas dans de telles synthèses « one-pot », où les impuretés générées dans les premières étapes ne sont pas éliminées.

Plus précisément, la présente invention concerne un procédé de préparation de bis-DMTD comprenant au moins les étapes réactionnelles suivantes :
a) réaction d'hydrazine N₂H₄ avec du sulfure de carbone CS₂, en milieu basique ;
b) acidification du milieu réactionnel ;
c) oxydation du milieu réactionnel ;
d) récupération et purification éventuelle du bis-DMTD formé.
ledit procédé étant réalisé sans isolement de DMTD intermédiaire.

L'étape a) est réalisée en phase aqueuse, par exemple dans de l'eau. L'utilisation d'une phase hydro-organique peut être envisagée, mais n'est pas préférée en raison des difficultés de traitement de ces effluents hydro-organiques et organiques en fin de réaction, et de leur impact sur l'environnement.

L'hydrazine utilisée peut être de tout type, et on préfère utiliser le monohydrate d'hydrazine N₂H₄, H₂O. De même, il n'existe aucune préconisation particulière quant à la nature du sulfure de carbone CS₂ utilisé, si ce n'est les précautions d'emploi habituelles liées à l'usage de ce produit.

Selon un mode de réalisation préféré, les matières premières utilisées dans le procédé de la présente invention comprennent une teneur en métaux dissous la plus faible possible, voire sont exemptes de métaux dissous. Selon un mode de réalisation plus particulièrement préféré, les teneurs en métaux dissous dans les matières premières sont inférieures à 300 ppm, avantageusement inférieures à 200 ppm, et très avantageusement inférieure à 100 ppm.

Le ratio molaire sulfure de carbone/hydrazine (CS₂/N₂H₄), peut varier dans de grandes proportions, et est avantageusement compris entre 1,8 et 4,0, de préférence entre 2,0 et 3,0, de préférence encore entre 2,0 et 2,5, de manière tout à fait préférée entre environ 2,2 et 2,4.

Cette première étape de réaction entre l'hydrazine et le sulfure de carbone est réalisée en milieu basique, par exemple en présence d'une base forte, minérale ou organique, de préférence minérale. De préférence encore, la base minérale est un hydroxyde d'alcalin ou d'alcalino-terreux, l'hydroxyde de sodium étant préféré, notamment en raison de son faible coût.

La quantité de base dans le milieu réactionnel peut également varier dans de grandes proportions. Généralement, le ratio molaire base/hydrazine est compris entre 0,8 et 1,5, de préférence entre 1,0 et 1,2 environ.

Selon un aspect préféré, le sulfure de carbone est ajouté lentement à la solution aqueuse basique d'hydrazine à une température comprise entre 20°C et 45°C de préférence, par exemple aux environs de 40°C.

Après addition du sulfure de carbone, la température réactionnelle peut être ajustée selon notamment la cinétique de réaction souhaitée. Ainsi, la température réactionnelle peut être fixée à une valeur comprise entre 0°C et 100°C, par exemple aux environs de 98°C.

Cette première étape est généralement conduite à pression atmosphérique, mais il n'est pas exclu d'opérer sous pression, ou encore sous léger vide. De manière particulièrement avantageuse, cette étape, ainsi que l'ensemble du procédé est conduit sous atmosphère inerte, par exemple sous azote.

La réaction entre l'hydrazine et le sulfure de carbone conduit à la formation d'un sel de DMTD, non isolé, avec dégagement de sulfure de di-hydrogène, qui peut être avantageusement piégé, selon toute méthode connue de l'homme du métier, et par exemple dans un piège contenant une solution aqueuse d'hydroxyde de sodium.

Après refroidissement du milieu réactionnel issu de l'étape a), le sel de DMTD est acidifié, au sein du même réacteur de synthèse, en DMTD.

Cette étape b) d'acidification est conduite sous l'action d'un acide, généralement un acide fort, minéral ou organique, de préférence minéral, tel que par exemple l'acide sulfurique, l'acide nitrique ou l'acide chlorhydrique, de préférence l'acide sulfurique.

On utilisera de préférence une solution d'acide dans l'eau pour la réalisation de cette étape. La concentration en acide peut varier dans de grandes proportions, selon la nature de la base utilisée à l'étape a), et selon le pH du milieu réactionnel.

De préférence, la quantité d'acide ajouté dans le milieu réactionnel est telle que le pH du milieu soit inférieur à 5, de préférence inférieur à 4, plus spécifiquement compris entre 0 et 4.

Selon une variante, la quantité d'acide ajoutée est nécessaire et suffisante pour acidifier le milieu réactionnel tout en maintenant le DMTD formé sous forme de mono-sel de DMTD, comprenant une fonction -SH libre, et une fonction -SX, où X représente le contre-ion de la base utilisée à l'étape a), comme illustré par la formule suivante :

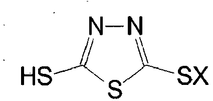

À titre d'exemple, lorsque la base utilisée à l'étape a) est l'hydroxyde de sodium, X représente l'atome de sodium Na. L'utilisation du mono-sel ci-dessus dans l'étape c) d'oxydation en bis-DMTD définie plus bas présente les avantages décrit dans la demande de brevet US 2006/0168741, permettant d'améliorer la sélectivité de formation du dimère de DMTD en diminuant les risques de formation de polymères supérieurs et de dimères cycliques.

L'étape b) est généralement réalisée à température ambiante, et il peut être nécessaire, mais non obligatoire, de refroidir le milieu réactionnel, pour maintenir la température au voisinage de l'ambiante, pendant l'ajout de la solution acide.

À l'issue de cette étape d'acidification, le milieu réactionnel, contenant principalement du DMTD majoritairement en suspension dans l'eau, et une partie en solution dans l'eau, est engagé, toujours dans le même réacteur, sans isolation ni séchage du DMTD intermédiaire, dans l'étape c) d'oxydation du DMTD en bis-DMTD.

Pour ce faire, on ajoute au milieu réactionnel une quantité d'un agent oxydant qui peut être de tout type utilisé généralement dans le domaine, et par exemple choisi parmi les peroxydes, tels que le peroxyde d'hydrogène (eau oxygénée), les peracides organiques ou minéraux, et notamment l'acide peracétique, et les mélanges d'entre eux ou avec des acides forts, par exemple les mélanges peroxyde d'hydrogène/acides forts tels que l'acide sulfurique ou chlorhydrique.

On préfère utiliser le peroxyde d'hydrogène en tant qu'agent oxydant. L'utilisation d'acide peracétique libère, après réaction, de l'acide acétique dans les effluents, augmentant alors la charge DCO. Toutefois cette charge DCO due à la présence d'acide acétique peut être facilement abattue par traitement classique des effluents (par exemple station d'épuration). Ce traitement d'effluents chargés en acide acétique est bien entendu beaucoup plus aisé que le traitement des effluents chargés en DMTD observés avec les procédés classiques connus de l'art antérieur.

L'agent oxydant préféré pour le procédé de la présente invention est l'eau oxygénée (peroxyde d'hydrogène) à une concentration comprise entre 30% et 70%, par exemple environ 35% en poids.

Le ratio molaire agent oxydant/DMTD est généralement compris entre 0,35 : 1 et 0,65 : 1, de préférence le ratio molaire est compris entre 0,45 : 1 et 0,55 : 1. Un ratio molaire en deçà de 0,45 : 1 peut être insuffisant pour assurer une conversion la plus complète possible du DMTD en bis-DMTD sans générer de sous-produits, tandis qu'un ratio molaire au-delà de 0,55 : 1, peut conduire à la formation de sous-produits non souhaités, tels que des produits de polymérisations du DMTD.

Pour l'étape d'oxydation, l'agent oxydant est ajouté au milieu réactionnel issu de l'étape b) et la réaction d'oxydation est conduite à une température généralement comprise entre 10°C et 100°C, de préférence entre 35°C et 65°C, et de manière tout particulièrement préférée entre 50°C et 55°C.

Comme pour les étapes précédentes, l'étape d'oxydation est de préférence et avantageusement conduite sous pression atmosphérique, de manière préférée sous atmosphère inerte.

Le Bis-DMTD formé se présente sous la forme de particules fines. Il peut également être avantageux d'ajouter au milieu réactionnel, pendant ou après la réaction d'oxydation, un agent de mise en oeuvre, permettant une récupération plus aisée du précipité de bis-DMTD car permettant de limiter le caractère pulvérulent de la poudre finale et donc de faciliter son utilisation.

Les agents de mise en oeuvre peuvent être de tout type, et sont bien connus de l'homme du métier. Des agents de mise en oeuvre particulièrement appropriés sont par exemple les huiles, avantageusement de type huiles naphténiques ou parafféniques, en particulier l'huile « 85 NEUTRAL SOLVENT » de la société Total.

La quantité d'agent de mise oeuvre peut varier dans de grandes proportions et l'homme du métier saura adapter cette quantité aux besoins spécifiques résultant de la synthèse réalisée. En règle générale, la quantité d'agent de mise en oeuvre est comprise entre 0,01 % et 10% en poids par rapport à la masse attendue de bis-DMTD formé, de préférence, et notamment lorsque l'agent de mise en oeuvre est une huile comme indiqué précédemment, la quantité pondérale ajoutée est comprise entre 0,1% et 5% de la masse attendue de bis-DMTD, de préférence encore entre 1 % et 3%.

Le procédé selon la présente invention peut avantageusement être conduit en présence d'au moins un tensio-actif, de préférence non-ionique, tel que par exemple le Tergitol™ 15-S-5 de la société Dow Chemicals.

La quantité de tensio-actif est généralement comprise entre 0,01% en poids et 10% en poids, de préférence entre 0,1% et 5%, de préférence encore entre 0,1 et 1%, de la masse attendue de bis-DMTD.

Le produit final attendu, sous forme de précipité dans le milieu réactionnel, phase aqueuse voire hydro-organique, est ainsi récupéré selon les méthodes classiques connues dans le domaine, par exemple choisies parmi une ou plusieurs des opérations suivantes : décantation, filtration, centrifugation, essorage, pressage, et autres.

Le bis-DMTD obtenu sous forme solide peut être ensuite lavé, avantageusement à l'eau, notamment dans le but d'éliminer les impuretés hydrosolubles, telles que les sulfates et les hydrogénosulfates. Le bis-DMTD peut être, si on le souhaite ou si cela est nécessaire, purifié, selon toute méthode classique de purification connue de l'homme du métier, et par exemple par recristallisation dans un ou plusieurs solvants appropriés.

Enfin, le bis-DMTD est avantageusement séché, par exemple sous vide ou sous courant d'air, éventuellement en association avec une température comprise avantageusement entre 50°C et 150°C, de préférence entre 60°C et 110°C, de préférence encore entre 70°C et 90°C, avant conditionnement éventuel, notamment pour le stockage et le transport du produit fini.

Le procédé de la présente invention présente l'avantage d'engager dans la réaction d'oxydation à la fois le DMTD solide en suspension et le DMTD soluble dans l'eau, avec un taux de conversion quasi total du DMTD, sans rejet ou seulement avec de très faibles quantités de DMTD rejetées dans les effluents issus de la réaction.

De plus, le bis-DMTD étant quasiment insoluble dans l'eau, les effluents ainsi générés lors du procédé de l'invention, y compris les eaux-mères résultant de la récupération ne contiennent ni DMTD ni bis-DMTD, ou seulement des traces de ces produits : il en résulte que la quantité de matières organiques dans ces effluents (DCO), est très faible, généralement inférieure à 10g/L, plus généralement inférieure à 5 g/L, voire inférieure à 2 g/L, contrairement aux procédés classiques réalisés en deux étapes dont la teneur en DCO dans les effluents s'élève généralement à des valeurs supérieures à 15 g/L.

La demande chimique en oxygène (D.C.O.) de l'eau est définie comme étant la masse volumétrique en oxygène équivalente à la masse en dichromate de sodium consommée dans des conditions opérationnelles données pour l'oxydation des matières oxydables dans l'eau. La mesure de la DCO peut être effectuée selon toute méthode connue de l'homme du métier, en particulier selon la méthode de référence ISO 15705:2002.

Le procédé de l'invention permet donc d'éviter le rejet d'effluents chargés en DMTD, et par conséquent une perte de l'intermédiaire de synthèse. Ceci conduit à des gains de rendements non négligeables par rapport aux synthèses de l'art antérieur, la totalité du DMTD formée en tant qu'intermédiaire étant utilisée dans la réaction d'oxydation conduisant au bis-DMTD.

Ainsi, et selon les concentrations du milieu réactionnel mises en oeuvre, des gains en rendement, basés sur le nombre de moles de bis-DMTD formées par rapport au nombre de moles d'hydrazine engagées au départ, sont de l'ordre de 5% à 10%, voire supérieurs.

Le procédé selon la présente invention est par conséquent tout à fait adapté à une production industrielle, et ceci d'autant plus ledit procédé « one-pot » permet d'obtenir le bis-DMTD avec une pureté élevée, généralement supérieure à 96%, plus généralement supérieure à 98%, voire supérieure à 99%.

Le bis-DMTD préparé selon le procédé de l'invention trouve des applications dans de nombreux domaines, notamment en raison en raison de ses propriétés anti-usure, de sa bonne résistance aux températures et pressions élevées.

Ainsi le bis-DMTD peut avantageusement être utilisé comme agent anti-usure dans les lubrifiants, par exemple pour les huiles de moteurs, les fluides caloporteurs, les huiles de travail des métaux, et de manière plus générale dans tout type d'huile susceptibles d'être soumises à des températures et/ou des pressions élevées.

Le bis-DMTD présente également des propriétés anti-oxydantes et peut ainsi être utilisé dans un grand nombre d'applications, telles que par exemple celles définies ci-dessus, mais aussi les revêtements, notamment peintures, laques, vernis, et autres produits filmogènes, notamment à base aqueuse, et en particulier pour le revêtement de pièces métalliques (bâtiment, huisseries, quincaillerie, serrurerie), et dans la production de dispositifs électro-chimiques, tels que piles (chimiques rechargeables, à combustible, et autres), accumulateurs, condensateurs, cellules d'électrolyse ou de galvanoplastie, pour ne citer que quelques unes d'entre elles, et sans apporter aucune quelconque limitation.

La présente invention est maintenant illustrée au moyen de l'exemple qui suit et qui ne présente aucun but limitatif au regard de la portée de la présente invention, portée par ailleurs définie par les revendications annexées.

### Exemple de synthèse de bis-DMTD :

### Étape a)

On ajoute dans un réacteur vitrifié ou en acier inox de 16 m³, préalablement inerté à l'azote, et muni d'un système d'agitation, 4000 litres d'eau, 800 kg d'hydrate d'hydrazine monohydratée et 1300 kg de solution aqueuse d'hydroxyde de sodium à 50% en poids. La température monte à environ 30°C.

On ajoute ensuite, en 10 heures environ, 2900 kg de sulfure de carbone à une température comprise entre 36°C et 40°C. Le sulfure de di-hydrogène libéré pendant la réaction est piégé par absorption/réaction dans une solution aqueuse d'hydroxyde de sodium.

On chauffe progressivement le milieu réactionnel à environ 98°C et on réalise un stripping à l'azote à cette température pendant 5 heures. On refroidit ensuite le milieu réactionnel à 20°C, puis on ajoute 2000 L d'eau.

### Étape b)

Toujours sous agitation et sous azote, on ajoute 2200 kg d'acide sulfurique à 42%, tout en maintenant la température à 20°C.

### Étape c)

On ajoute alors 770 kg de peroxyde d'hydrogène (H₂O₂) aqueux à 35%, en maintenant la température entre 50°C et 55°C, puis on maintient l'agitation pendant 2 heures à 50°C.

### Étape d)

On ajoute alors 36 kg d'huile 85 NEUTRAL SOLVENT de la société Total, puis on filtre le produit sur une essoreuse ; le produit est lavé à l'eau, puis séché sous vide à 70°C. On récupère 2240 kg de bis-DMTD, de point de fusion 162-164°C et de pureté 98 % (rendement = 92%).

La DCO (demande chimique en oxygène) des eaux-mères est mesurée avec un spectrophotomètre DR/5000 (Hach-Lange), après filtration du bis-DMTD. La DCO mesurée est inférieure ou égale à 5 g/L.

La faible toxicité des effluents générés par le procédé de la présente invention, ainsi que les bons rendements observés, rendent ledit procédé tout à fait approprié pour une fabrication de bis-DMTD à l'échelon industriel.

## Revendications

1. Procédé de préparation de bis-DMTD comprenant au moins les étapes suivantes :
a) réaction d'hydrazine N₂H₄ avec du sulfure de carbone CS₂, en milieu basique ;
b) acidification du milieu réactionnel ;
c) oxydation du milieu réactionnel ;
d) récupération et purification éventuelle du bis-DMTD formé,
ledit procédé étant réalisé sans isolement de DMTD intermédiaire.

2. Procédé selon la revendication 1, dans lequel le ratio molaire CS₂/N₂H₄ est compris entre 1,8 et 4,0, de préférence entre 2,0 et 3,0, de préférence encore entre 2,0 et 2,5, de manière tout à fait préférée entre environ 2,2 et 2,4.

3. Procédé selon la revendication 1 ou la revendication 2, dans lequel l'étape a) est réalisée en phase aqueuse.

4. Procédé selon l'une quelconque des revendications précédentes, dans lequel l'étape a) est réalisée en milieu basique, de préférence en présence d'une base forte, de préférence encore en présence d'un hydroxyde d'alcalin ou d'alcalino-terreux.

5. Procédé selon l'une quelconque des revendications précédentes, dans lequel le ratio molaire base/hydrazine est compris entre 0,8 et 1,5, de préférence entre 1,0 et 1,2 environ.

6. Procédé selon l'une quelconque des revendications précédentes, dans lequel l'étape a) est conduite en présence d'un tensio-actif, de préférence non-ionique.

7. Procédé selon l'une quelconque des revendications précédentes, dans lequel, l'étape d'acidification est conduite sous l'action d'un acide, de préférence un acide fort minéral ou organique, de préférence encore un acide fort minéral, de préférence l'acide sulfurique.

8. Procédé selon l'une quelconque des revendications précédentes, dans lequel l'étape c) d'oxydation est réalisée par ajout d'un agent oxydant au milieu réactionnel, ledit agent oxydant étant choisi parmi les peroxydes, tels que le peroxyde d'hydrogène, les peracides organiques ou minéraux, et notamment l'acide peracétique, et les mélanges d'entre eux ou avec des acides forts, par exemple les mélanges peroxyde d'hydrogène/acides forts tels que l'acide sulfurique ou chlorhydrique, l'agent oxydant étant de préférence le peroxyde d'hydrogène.

9. Procédé selon l'une quelconque des revendications précédentes, dans lequel le ratio molaire agent oxydant/DMTD est compris entre 0,35 :1 et 0,65 :1, de préférence le ratio molaire est compris entre 0,45 :1 et 0,55 :1.

10. Procédé selon l'une quelconque des revendications précédentes, dans lequel un agent de mise en œuvre est ajouté au milieu réactionnel au cours de l'étape d), ledit agent de mise en œuvre étant de préférence une huile, avantageusement de type huile naphténique ou paraffénique.

11. Procédé selon l'une quelconque des revendications précédentes, dans lequel le bis-DMTD récupéré sous forme solide est lavé à l'eau, puis avantageusement séché à une température comprise entre 50°C et 150°C, de préférence entre 60°C et 110°C, de préférence encore entre 70°C et 90°C.

12. Procédé selon l'une quelconque des revendications précédentes, dans lequel la quantité de matières organiques dissoutes dans les effluents de réaction est très faible et conduit à une valeur de Demande Chimique en Oxygène (DCO) inférieure à 10 g/L, plus généralement inférieure à 5 g/L, voire inférieure à 2 g/L.

## Patentansprüche

1. Verfahren zur Herstellung von Bis-DMTD, umfassend mindestens die folgenden Schritte:
a) Reaktion von Hydrazin N₂H₄ mit Kohlenstoffdisulfid CS₂ in basischem Medium;
b) Ansäuerung des Reaktionsmediums;
c) Oxidation des Reaktionsmediums;
d) Gewinnung und eventuelle Aufreinigung des gebildeten Bis-DMTD,
wobei das Verfahren ohne Isolierung von intermediärem DMTD durchgeführt wird.

2. Verfahren nach Anspruch 1, wobei das CS₂/N₂H₄-Molverhältnis zwischen 1,8 und 4,0, vorzugsweise zwischen 2,0 und 3,0, mehr bevorzugt zwischen 2,0 und 2,5, besonders bevorzugt zwischen ungefähr 2,2 und 2,4 liegt.

3. Verfahren nach Anspruch 1 oder Anspruch 2, wobei der Schritt a) in wässriger Phase durchgeführt wird.

4. Verfahren nach einem der vorhergehenden Ansprüche, wobei der Schritt a) in basischem Medium, vorzugsweise in Gegenwart einer starken Base, mehr bevorzugt in Gegenwart eines Alkali- oder Erdalkalihydroxids durchgeführt wird.

5. Verfahren nach einem der vorhergehenden Ansprüche, wobei das Base/Hydrazin-Molverhältnis zwischen 0,8 und 1,5, vorzugsweise zwischen ungefähr 1,0 und 1,2 liegt.

6. Verfahren nach einem der vorhergehenden Ansprüche, wobei der Schritt a) in Gegenwart eines Tensids, vorzugsweise nichtionisch, vorgenommen wird.

7. Verfahren nach einem der vorhergehenden Ansprüche, wobei der Ansäuerungsschritt unter Einwirkung einer Säure, vorzugsweise einer starken anorganischen oder organischen Säure, mehr bevorzugt einer starken anorganischen Säure, vorzugsweise Schwefelsäure vorgenommen wird.

8. Verfahren nach einem der vorhergehenden Ansprüche, wobei der Oxidationsschritt c) durch Zugabe eines Oxidationsmittels zu dem Reaktionsmedium durchgeführt wird, wobei das Oxidationsmittel aus Peroxiden, wie Wasserstoffperoxid, organischen oder anorganischen Persäuren und insbesondere Peressigsäure und Gemischen dieser oder mit starken Säuren, beispielsweise Gemische von Wasserstoffperoxid/starken Säuren, wie Schwefelsäure oder Salzsäure, ausgewählt ist, wobei das Oxidationsmittel vorzugsweise Wasserstoffperoxid ist.

9. Verfahren nach einem der vorhergehenden Ansprüche, wobei das Oxidationsmittel/DMTD-Molverhältnis zwischen 0,35:1 und 0,65:1 liegt, vorzugsweise das Molverhältnis zwischen 0,45:1 und 0,55:1 liegt.

10. Verfahren nach einem der vorhergehenden Ansprüche, wobei ein Verarbeitungsmittel zu dem Reaktionsmedium während dem Schritt d) zugegeben wird, wobei das Verarbeitungsmittel vorzugsweise ein Öl, vorteilhafterweise vom naphthenischen oder paraffinischen Öl-Typ ist.

11. Verfahren nach einem der vorhergehenden Ansprüche, wobei das in fester Form gewonnene Bis-DMTD mit Wasser gewaschen wird, dann vorteilhafterweise bei einer Temperatur, die zwischen 50 °C und 150 °C, vorzugsweise zwischen 60 °C und 110 °C, mehr bevorzugt zwischen 70 °C und 90 °C liegt, getrocknet wird.

12. Verfahren nach einem der vorhergehenden Ansprüche, wobei die Menge an gelösten organischen Stoffen in den Reaktionsausflüssen sehr gering ist und zu einem Wert des chemischen Sauerstoffbedarfs (CSB) von weniger als 10 g/L, allgemeiner weniger als 5 g/L, sogar weniger als 2 g/L führt.

## Claims

1. Method for preparing bis-DMTD, comprising at least the following steps:
a) reaction of hydrazine N₂H₄ with carbon disulfide CS₂, in a basic medium;
b) acidification of the reaction medium;
c) oxidation of the reaction medium;
d) recovery and optional purification of the bis-DMTD formed,
said method being performed without intermediate isolation of DMTD.

2. Method according to claim 1, wherein the CS₂/N₂H₄ molar ratio is between 1.8 and 4.0, preferably between 2.0 and 3.0, preferably again between 2.0 and 2.5, entirely preferably between approximately 2.2 and 2.4.

3. Method according to claim 1 or claim 2, wherein step a) is performed in aqueous phase.

4. Method according to any one of the preceding claims, wherein step a) is performed in a basic medium, preferably in the presence of a strong base, preferably again in the presence of an alkaline or alkaline-earth hydroxide.

5. Method according to any one of the preceding claims, wherein the base/hydrazine molar ratio is between 0.8 and 1.5, preferably between approximately 1.0 and 1.2.

6. Method according to any one of the preceding claims, wherein step a) is conducted in the presence of a surfactant, preferably non-ionic.

7. Method according to any one of the preceding claims, wherein the acidification step is conducted under the action of an acid, preferably a mineral or organic strong acid, preferably again a mineral strong acid, preferably sulfuric acid.

8. Method according to any one of the preceding claims, wherein the oxidation step c) is performed by adding an oxidizing agent to the reaction medium, said oxidizing agent being chosen from peroxides, such as hydrogen peroxide, organic or mineral peracids, and in particular peracetic acid, and mixtures with each other or with strong acids, for example mixtures of hydrogen peroxide and strong acids such as sulfuric or hydrochloric acid, the oxidizing agent preferably being hydrogen peroxide.

9. Method according to any one of the preceding claims, wherein the oxidizing agent/DMTD molar ratio is between 0.35:1 and 0.65:1, and preferably the molar ratio is between 0.45:1 and 0.55:1.

10. Method according to any one of the preceding claims, wherein an implementation agent is added to the reaction medium during step d), said implementation agent preferably being an oil, advantageously of the naphthene or paraffin oil type.

11. Method according to any one of the preceding claims, wherein the bis-DMTD recovered in solid form is washed with water, and then advantageously dried at a temperature of between 50°C and 150°C, preferably between 60°C and 110°C, preferably again between 70°C and 90°C.

12. Method according to any one of the preceding claims, wherein the quantity of organic matter dissolved in the reaction effluents is very small and leads to a chemical oxygen demand (COD) value of less than 10 g/l, more generally less than 5 g/l, or even less than 2 g/l.
